## Europäisches Patentamt

## European Patent Office

## Office européen des brevets

(19)

(11) Veröffentlichungsnummer: **0 026 316**
**B1**

(12) **EUROPÄISCHE PATENTSCHRIFT**

(45) Veröffentlichungstag der Patentschrift:
26.05.82

(51) Int. Cl.³: **C 07 C 87/60**, C 07 C 85/145,
C 07 C 87/54

(21) Anmeldenummer: **80104894.3**

(22) Anmeldetag: **16.08.80**

(54) **Verfahren zur Herstellung von 4-Nitroso-diphenylamin.**

(30) Priorität: **05.09.79 DE 2935775**

(43) Veröffentlichungstag der Anmeldung:
**08.04.81 Patentblatt 81/14**

(45) Bekanntmachung des Hinweises auf die Patenterteilung:
**26.05.82 Patentblatt 82/21**

(84) Benannte Vertragsstaaten:
**BE DE FR GB IT NL**

(56) Entgegenhaltungen:
**DE-A-2 211 341**

(73) Patentinhaber: **BAYER AG, Zentralbereich Patente, Marken und Lizenzen, D-5090 Leverkusen 1, Bayerwerk (DE)**

(72) Erfinder: **Wedemeyer, Karlfried, Dr., Bilharzstrasse 7, D-5000 Koeln 80 (DE)**
Erfinder: **Kienitz, Lutz, Dr., Forststrasse 2, D-5060 Bergisch-Gladbach-1 (DE)**

## Verfahren zur Herstellung von 4-Nitroso-diphenylamin

Die vorliegende Erfindung betrifft ein Verfahren zur Herstellung von 4-Nitroso-diphenylamin durch Umlagerung von N-Nitroso-diphenylamin nach Fischer-Hepp in einem inerten, mit Wasser nicht mischbaren Lösungsmittel unter Einwirkung alkoholischer Salzsäure und Aufarbeitung der als Reaktionsgemisch anfallenden Suspension von 4-Nitroso-diphenylamin-hydrochlorid mit wässrigen Alkalien.

4-Nitroso-diphenylamin ist ein wichtiges Zwischenprodukt für die Herstellung von Alterungsschutzmitteln für Kautschuk, von Farbstoffen oder von Kompositionen für den Korrosionsschutz. Die Herstellung erfolgt fast ausnahmslos durch N-Nitrosierung von Diphenylamin und Umlagerung des entstandenen N-Nitroso-diphenylamins nach Fischer Hepp. Für die Umlagerung ist eine ganze Reihe von Varianten beschrieben. Im allgemeinen wird die N-Nitroso-Verbindung in einem inerten, mit Wasser nicht mischbaren Lösungsmittel vorgelegt und unter Einwirkung methanolischer bzw. alkoholischer Salzsäure umgelagert. Es fällt zunächst eine Suspension des 4-Nitroso-diphenylamin-hydrochlorids an. Da 4-Nitroso-diphenylamin in seiner tautomeren Form, dem p-Benzochinon-aniloxim eine Säure ist, kann es mit wässrigem Alkali in Form seines Alkalisalzes extrahiert werden. Man erreicht so eine Abtrennung von nicht umgelagertem N-Nitroso-diphenylamin, von Diphenylamin als Entnitrosierungsprodukt und von weiteren Zersetzungsprodukten, die – da sie alkaliunlöslich sind – in der organischen Phase verbleiben. Durch Neutralisation mit verdünnten Mineralsäuren kann man das freie 4-Nitroso-diphenylamin erhalten, die wässrig-alkalische Lösung kann aber auch direkt zur Reduktion zu 4-Amino-diphenylamin eingesetzt werden.

Als Beispiele für diese Arbeitsweise seien die US-Patentschriften 3 748 362 und 3 728 392 oder die DE-OS 2 654 936 genannt. Die in den angeführten Beispielen verwendeten, mit Wasser nicht mischbaren Lösungsmittel besitzen gewisse Nachteile. Aromatische Kohlenwasserstoffe wie Benzol, Toluol u.ä. als Lösungsmittel für das N-Nitroso-diphenylamin führen infolge ihrer geringen Dichte zu grobkörnigen, leicht sedimentierenden Suspensionen des 4-Nitroso-diphenylamin-hydrochlorids. Ausserdem neigen die Partikel dieser Suspension zum Verbacken. Solche Suspensionen sind nur schwer pumpfähig, so dass eine störungsfreie kontinuierliche Fahrweise in mehrstufigen Kaskaden nicht gewährleistet ist. So wird in der DE-OS 2 654 936 die kontinuierliche Umlagerung nur in Trichlorethylen beschrieben. In der DE-OS 2 211 341 wird die Schwierigkeit im Umgang mit 4-Nitroso-diphenylamin-hydrochlorid-Suspensionen umgangen, indem durch entsprechenden Einsatz der Lösungsmittelmenge für eine vollständige Lösung gesorgt wird. Diese Fahrweise ist jedoch verbunden mit hohen Aufwandmengen an Chlorwasserstoff, was bei der nachfolgenden Neutralisation zu einem verstärkten Salzanfall führt.

Arbeitet man in Methanol oder anderen Alkoholen ohne ein zusätzliches Lösungsmittel (z.B. US-PS 2 046 356 oder US-PS 3 429 924), so gestaltet sich die Wiedergewinnung des Alkohols problematisch, und der Vorteil der Abtrennung des nicht umgelagerten N-Nitroso-diphenylamins und der Zersetzungsprodukte geht verloren.

Chlorbenzol als Colösungsmittel für das N-Nitroso-diphenylamin hat den Nachteil, dass infolge der ungünstigen Dichteverhältnisse die Trennung zwischen der organischen und der wässrig-alkalischen Phase langwierig ist. Trichlorethylen wäre an sich das Lösungsmittel der Wahl. Man erhält sowohl bei kontinuierlicher als auch diskontinuierlicher Fahrweise hohe Ausbeuten, die zunächst anfallende 4-Nitroso-diphenylamin-hydrochlorid-Suspension ist feinkörnig und kann gepumpt werden (DE-OS 2 654 936; N.V. Martynov, L.V. Muratova, V.F. Pivovarova, Khim. Prom.-st' 1976, (9), 657–9). Da sich Trichlorethylen jedoch im Tierversuch als cancerogen erwiesen hat (siehe Registry of Toxic Effects of Chemical Substances, NIOSH Vol. II, (1977), besteht die Notwendigkeit, Trichlorethylen durch ein anderes Lösungsmittel mit den Vorteilen des Trichlorethylens aber ohne dessen Toxikologische Vorbehalte zu ersetzen.

Es wurde nun ein Verfahren zur Umlagerung von N-Nitroso-diphenylamin zu 4-Nitroso-diphenylamin nach Fischer-Hepp in einem inerten, mit Wasser nicht mischbaren Lösungsmittel unter Einwirkung alkoholischer Salzsäure und Aufarbeitung der als Reaktionsgemisch anfallenden 4-Nitroso-diphenylamin-hydrochlorid-Suspension durch Extraktion mit wässrigen Alkalien gefunden, bei dem als mit Wasser nicht mischbaren Lösungsmittel o-Dichlorbenzol eingesetzt wird.

Die Reihenfolge der Zugabe des Ausgangsmaterials ist beliebig. Man kann sowohl die organische Lösung des N-Nitrosoproduktes der alkoholischen Salzsäurelösung zugeben, als auch umgekehrt.

Man legt bevorzugt in dem erfindungsgemässen Verfahren das N-Nitroso-diphenylamin in o-Dichlorbenzol vor. Die Konzentrationsverhältnisse sind dabei nicht kritisch. Sie sind nach unten durch Zweckmässigkeitsüberlegungen begrenzt, nach oben hin durch die Löslichkeit des N-Nitroso-diphenylamins. Zu verdünnte Lösungen führen zu einer ungünstigen Raum-Zeit-Ausbeute. Bevorzugt sind 5–50 Gew.%ige Lösungen.

Der zur Umlagerung erforderliche saure Katalysator wird in Form alkoholischer Salzsäure eingesetzt. Als Alkohole kommen Alkanole vom Typ $C_nH_{2n+1}OH$ mit n = 1–6 oder Cycloalkanole vom Typ $C_{n_1}H_{2n_1-1}OH$ mit $n_1$ = 5 oder 6 in Frage. Setzt man mit Wasser mischbare Alkanole ein, so wählt man zweckmässigerweise solche, deren Siedepunkt niedriger als der des Wassers liegt, so dass eine leichte Wiedergewinnung aus der wässrigen Phase gewährleistet ist. Bevorzugt werden Methanol, Ethanol oder Isopropanol eingesetzt, besonders bevorzugt Methanol. Die Menge des zur Umlagerung nötigen sauren Katalysators beträgt mindestens 100 Mol%, bezogen auf das eingesetzte N-Nitroso-diphenylamin, und ist nach oben hin nur durch die Wirtschaftlichkeit des Verfahrens begrenzt. Bevorzugt sind 100 – 500 Mol%. Die als saurer Katalysator verwendete alkoholische Salzsäure wird in Form von 5 Gew.%igen bis gesättigten Lösungen in den oben genannten Alkoholen eingesetzt. Die Reaktionstemperatur kann zwischen 0° und 50°C betragen, wobei nach oben eine Grenze durch die Zersetzlichkeit des 4-Nitroso-diphenylamin-hydrochlorids gesetzt ist. Die Reaktionszeit wird zwischen 1 und 10 h gewählt, wobei auch hier eine Verlängerung infolge der Zersetzlichkeit des 4-Nitroso-diphenylamin-hydrochlorids nachteilig ist. Da das 4-Nitroso-diphenylamin in seiner tautomeren Form – dem p-Benzochinolaniloxim – eine Säure ist, kann es auch aus dem nach der Umlagerung vorliegenden Reaktonsgemisch mittels wässriger Alkalien extrahiert werden. Als wässrige Alkalien können Natronlauge, Kalilauge, Sodalösung eingesetzt werden. Bevorzugt verwendet man Natronlauge. Die Konzentration ist nicht kritisch und kann 5–50 Gew.% betragen. Die Alkalimenge muss mindestens zur Neutralisation der zur Umlagerung verwendeten sauren Katalysatoren und zur Überführung des 4-Nitroso-diphenylamins in sein Alkalisalz ausreichen und beträgt bevorzugt 200–1000 Mol%, bezogen auf die eingesetzte Menge des N-Nitroso-diphenylamins. Der wässrig-alkalische Extrakt wird abgetrennt und kann direkt in die Reduktion zu 4-Amino-diphenylamin eingesetzt werden. Aus dem wässrig-alkalischen Extrakt kann aber auch das freie 4-Nitroso-diphenylamin durch Neutralisation mit verdünnten Mineralsäuren freigesetzt werden.

Das erfindungsgemässe Verfahren gestattet die Herstellung von 4-Nitroso-diphenylamin in hohen Ausbeuten und in vorzüglicher Reinheit. Das erfindungsgemässe Verfahren vermeidet vor allem den Umgang mit dem in den bekannten Varianten bevorzugten, aber im Tierversuch als cancerogen erkannten Trichlorethylen.

Die Verwendung von o-Dichlorbenzol erbringt ausgezeichnete Ergebnisse bei diskontinuierlicher Fahrweise, gestattet aber insbesondere auch eine kontinuierliche Fahrweise in mehrstufigen Kaskaden, denn die Suspension des zunächst anfallenden 4-Nitroso-diphenylamin-hydrochlorids in o-Dichlorbenzol ist ebenso wie die in Trichlorethylen gut pumpbar. Es ist ferner nicht erforderlich, das N-Nitroso-diphenylamin in isolierter Form einzusetzen. Vielmehr kann man die N-Nitrosierung des Diphenylamins in dem Zweiphasensystem wässrige Schwefelsäure/o-Dichlorbenzol durchführen und die anfallende Lösung des N-Nitroso-diphenylamins in der organischen Phase direkt in die Umlagerung einsetzen. Das erfindungsgemässe Verfahren gestattet die Umlagerung bereits bei niedrigen Aufwandmengen an Chlorwasserstoff, so dass der Salzanfall bei der Aufarbeitung gering ist.

Das Verfahren soll anhand von Beispielen erläutert werden.

Beispiel 1

In einen Kolben, versehen mit Rührer, Tropftrichter, Innenthermometer und Rückflusskühler, legt man eine 20 Gew.%ige Lösung von 99 g = 0,5 M N-Nitroso-diphenylamin in o-Dichlorbenzol vor und tropft bei 20°C in 0,5 h 0,7 M = 25,6 g Chlorwasserstoff (entsprechend 140 Mol%, bezogen auf das N-Nitroso-diphenylamin) in Form einer 35 Gew.%igen methanolischen Salzsäure zu. Man rührt 8 h nach, gibt das Reaktionsgemisch zu einer Lösung von 80 g NaOH in 720 ml Wasser, rührt 0,5 h nach, trennt die organische Phase ab und schüttelt sie mit 200 ml 10 Gew.%iger Natronlauge aus. Aus den vereinigten wässrig-alkalischen Extrakten erhält man durch Neutralisation mit 20 Gew.%iger Schwefelsäure 94 g ≙ 95% 4-Nitroso-diphenylamin vom Schmp. 145–7°C. Die organische Phase enthält noch 0,4 g ≙ 0,4% 4-Nitroso-diphenylamin, 2,7 g ≙ 2,7% nicht umgelagertes N-Nitroso-diphenylamin und 1,2 g ≙ 1,4% Diphenylamin (Entnitrosierungsprodukt). Damit beläuft sich die Ausbeute – bezogen auf den Umsatz des N-Nitroso-diphenylamins – auf 97,6%.

Die Analytik erfolgt mittels Hochdruckflüssigkeitschromatographie gegen o-Terphenyl als innerem Standard, zur Kontrolle wird das 4-Nitroso-diphenylamin mit Tetrabutylammoniumhydroxid in Isopropanol potentiometrisch titriert.

Beispiel 2

In eine Kaskade aus 4 hintereinandergeschalteten doppelwandigen, separat gekühlten Rührgefässen pumpt man simultan eine 22 Gew.%ige Lösung von N-Nitroso-diphenylamin in o-Dichlorbenzol und eine 35 Gew.%ige methanolische Salzsäure so ein, dass das Molverhältnis von HCl zu N-Nitroso-diphenylamin 1,4 beträgt und die Verweilzeit pro Gefäss bei 3,11 h liegt. Die Temperatur wird bei 20°C gehalten. Nachdem sich in dem System ein quasistationärer Zustand eingestellt hat, nimmt das Reaktionsgemisch in 2- oder 4-Stundenportionen ab. Dazu wird das Reaktionsgemisch aus dem letzten Gefäss in ein Auffanggefäss, gefüllt mit überschüssiger 10 Gew.%iger Natronlauge, geleitet. Man trennt die organische Phase ab, schüttelt sie mit 10 Gew.%iger Natronlauge aus und neutralisiert die vereinigten wässrig-alkalischen Lösungen mit 20%iger Schwefelsäure. Man erhält ein 4-Nitroso-diphenylamin mit einem Schmp. von 146–7°C, die Ausbeute beträgt 93%. In der organischen Phase sind noch ca. 5% nicht umgesetztes N-Nitroso-diphenylamin enthalten.

**Patentansprüche**

1. Verfahren zur Herstellung von 4-Nitroso-diphenylamin durch Umlagerung von N-Nitroso-diphenylamin nach Fischer-Hepp in einem inerten mit Wasser nicht mischbaren Lösungsmittel unter Einwirkung alkoholischer Salzsäure und Aufarbeitung der als Reaktionsgemisch anfallenden Suspension des 4-Nitroso-diphenylamin-hydrochlorids durch Extraktion mit wässrigen Alkalien, dadurch gekennzeichnet, dass man als mit Wasser nicht mischbares Lösungsmittel o-Dichlorbenzol einsetzt.

2. Verfahren nach Anspruch 1, dadurch gekennzeichnet, dass man das N-Nitroso-diphenylamin in Form von 5 Gew.%igen bis gesättigten Lösungen einsetzt.

3. Verfahren nach Ansprüchen 1 und 2, dadurch gekennzeichnet, dass das N-Nitroso-Diphenylamin in Form einer 5 bis 50 Gew.%igen Lösung eingesetzt wird.

4. Verfahren nach Ansprüchen 1–3, dadurch gekennzeichnet, dass der zur Umlagerung benötigte saure Katalysator in Form einer 5 Gew.%igen bis gesättigten alkoholischen Salzsäure eingesetzt wird.

5. Verfahren nach Ansprüchen 1–4, dadurch gekennzeichnet, dass der zur Umlagerung benötigte saure Katalysator in Mengen von 100–500 Mol%, bezogen auf eingesetztes N-Nitroso-diphenylamin, angewandt wird.

6. Verfahren nach Ansprüchen 1–5, dadurch gekennzeichnet, dass als Lösungsmittel für den für die Umlagerung nötigen sauren Katalysator Alkanole vom Typ $C_nH_{2n+1}OH$ oder Cycloalkanole vom Typ $C_{n_1}H_{2n_1-1}OH$, eingesetzt werden, wobei n Zahlen von 1–6 und $n_1$ Zahlen von 5 oder 6 darstellen.

7. Verfahren gemäss Ansprüchen 1–6, dadurch gekennzeichnet, dass als Lösungsmittel für den sauren Katalysator Methanol, Ethanol und/oder Isopropanol eingesetzt wird.

## Claims

1. A process for the preparation of 4-nitroso-diphenylamine by rearranging N-nitroso-diphenylamine according to Fischer-Hepp in an inert solvent which is immiscible with water, under the effect of alcoholic hydrochloric acid and working up the suspension of the 4-nitroso-diphenylamino-hydrochloride, produced as the reaction mixture by extraction using aqueous alkalis characterised in that o-dichlorobenzene is used as the water-immiscible solvent.

2. A process according to claim 1, characterised in that the N-nitroso-diphenylamine is used in the form of a solution with a content of from 5% by weight to saturated.

3. A process according to claims 1 and 2, characterised in that the N-nitroso-diphenylamine is used in the form of a from 5 to 50% by weight solution.

4. A process according to claims 1 to 3, characterised in that the acidic catalyst required for rearrangement is used in the form of a 5% by weight to saturated alcoholic hydrochloric acid.

5. A process according to claims 1 to 4, characterised in that the acidic catalyst required for rearrangement is used in quantities of from 100 to 500 mol%, based on the N-nitroso-diphenylamine.

6. A process according to claims 1 to 5, characterised in that as a solvent for the acidic catalyst required for rearrangement, alkanols of the type $C_nH_{2n+1}OH$ or cycloalkanols of the type $C_{n_1}H_{2n_1-1}OH$ are used, whereby n represents numbers from 1 to 6 and $n_1$ represents 5 or 6.

7. A process according to claims 1 to 6, characterised in that, as a solvent for the acidic catalyst, methanol, ethanol, and/or isopropanol is used.

## Revendications

1. Procédé de production de la 4-nitroso-diphénylamine par transposition de N-nitroso-diphénylamine selon Fischer-Hepp dans un solvant inerte, non miscible à l'eau, sous l'action d'une solution alcoolique d'acide chlorhydrique et traitement, par extraction à l'aide de substances alcalines aqueuses, de la suspension de chlorhydrate de 4-nitroso-diphénylamine obtenue comme mélange réactionnel, procédé caractérisé en ce qu'on utilise l'o-dichlorobenzène comme solvant non miscible à l'eu.

2. Procédé selon la revendication 1, caractérisé en ce qu'on utilise la N-nitroso-diphénylamine sous forme de solutions contenant de 5% en poids jusqu'à la saturation.

3. Procédé selon les revendications 1 et 2, caractérisé en ce qu'on utilise la N-nitroso-diphénylamine sous forme d'une solution contenant 5 à 50% en poids.

4. Procédé selon les revendications 1 à 3, caractérisé en ce qu'on utilise le catalyseur acide, nécessaire pour la transposition, sous forme d'acide chlorhydrique alcoolique contenant de 5% en poids jusqu'à la saturation.

5. Procédé selon les revendications 1 à 4, caractérisé en ce qu'on applique le catalyseur acide, nécessaire pour la transposition, en des quantités de 100 à 500 moles%, par rapport à la N-nitroso-diphénylamine utilisée.

6. Procédé selon les revendications 1 à 5, caractérisé en ce qu'on utilise, comme solvant du catalyseur acide nécessaire pour la transposition, des alcanols du type $C_nH_{2n+1}OH$ ou des cycloalcanols du type $C_{n_1}H_{2n_1-1}OH$, où n est un nombre valant 1 à 6 et $n_1$ un nombre valent 5 ou 6.

7. Procédé selon les revendications 1 à 6, caractérisé en ce qu'on utilise, comme solvant pour le catalyseur acide, le méthanol, l'éthanol et éventuellement ou en variants l'isopropanol.